Europäisches Patentamt

European Patent Office

Office européen des brevets

(11) Publication number: **0 446 600 A1**

# EUROPEAN PATENT APPLICATION

(21) Application number: 91101469.4

(51) Int. Cl.5: **A61L 9/01, A43B 17/10**

(22) Date of filing: 04.02.91

(30) Priority: 08.02.90 IT 1930990

(43) Date of publication of application:
18.09.91 Bulletin 91/38

(84) Designated Contracting States:
AT BE CH DE ES FR GB IT LI NL

(71) Applicant: ABOCA S.r.l.
Frazione Aboca
I-52037 Sansepolcro (Arezzo)(IT)

(72) Inventor: Valentino, Mercati
Via Piero della Francesca, 32
Citta' di Castello, Perugia(IT)

(74) Representative: Dr. Ing. A. Racheli & C.
Viale San Michele del Carso, 4
I-20144 Milano(IT)

(54) Product for sanitizing, adsorbing moisture and/or perfuming footwear, and related method for use.

(57) A product for sanitizing, adsorbing moisture and/or perfuming shoes and footwear in general, consisting of a mixture of aromatic and medicinal herbs, with or without sprinkling with perfumed essences, and of adsorbent substances such as silica gel, calcium chloride, sodium sulphate and the like, in sizes varying from 2 to 20 mm, enclosed in a pliable porous wrapping (3) to be inserted into the inside of a shoe so that it substantially adapts to the whole of its inner surface.

## PRODUCT FOR SANITIZING, ADSORBING MOISTURE AND/OR PERFUMING FOOTWEAR, AND RELATED METHOD FOR USE

The aim of the present invention is a product for sanitizing, adsorbing moisture and/or perfuming shoes and footwear in general, as well as a pack and a method for using this product.

Excessive perspiration of the feet causes the perspiration to be adsorbed by the material used to make the footwear (leather of various types, cloth etc.) even after a relatively short period of use, with consequent dampness of the shoe, formation of unpleasant odours and infestation by fungi and bacteria.

In order to limit the problems connected with perspiration of the feet, use is usually made of dusting powder, bactericides and deodorants applied to the foot itself, or of socks to be applied to the insole of the shoe.

These devices do not however eliminate excess moisture, which is adsorbed by the footwear, which thus becomes fertile ground for the growth of fungi and bacteria, due, among other things, to the temperature and poor ventilation of their environment.

The mineral salts contained in perspiration are deposited and tend to obstruct further the natural porousness present in the materials used for the production of the footwear, thus worsening even more the hygienic conditions of the footwear and feet.

The aim of this invention is to eliminate the drawbacks mentioned above, by proposing a product capable of adsorbing, during the period when the shoes are not being used, the perspiration emitted in the footwear by the wearer's feet, and which will eliminate fungi and bacteria present and possibly perfume the shoes also.

The product according to the invention consists of a mixture of aromatic and medicinal herbs or of any other natural product with a bactericidal and fungicidal action, and of adsorbent substances, such as silica gel, calcium chloride, sodium sulphate etc., in sizes varying from 2 to 20 mm.

Perfumed essences may be added to the above-mentioned mixture.

The following is a preferred composition according to the invention:
- 20% thyme, 10% cinnamon, 20% origanum, 10% mint, 20% lavender, 20% silica gel.

The product according to the invention is conveniently packed in pliable porous wrappers, of cloth for example, in different dimensions according to the size of the footwear, and adapted to the inner surface of the same. Alternatively these containers may be envelopes, small bags, pads and the like, which are introduced in appropriate number according to the size of the footwear and of the containers.

The product according to the invention thus carries out sanitization of the footwear through the action of the fungicidal, bactericidal and deodorizing herbs, and adsorbs the moisture present in the footwear by means of the adsorbent substances such as silica gel or other substances.

The product packed in the way described above can be inserted into the shoes when they are not in use, at night for instance, thus improving the hygienic conditions of the shoe cupboard.

The antiseptic and deodorizing function of the herbs or other natural and non-natural products will expire after about six months/one year, while the adsorbent substances, such as the silica gel, can be regenerated after a period of use varying from one to two months by placing the product near to a source of heat, so as to eliminate the moisture adsorbed by the substances. The product according to the invention therefore allows:
- improvement of the hygienic conditions of the feet;
- elimination of unpleasant odour of the footwear;
- improvement of the hygienic conditions of the shoe cupboard;
- lowering of the costs deriving from the quick replacement of prematurely deteriorated footwear;
- carrying out an antiseptic function.

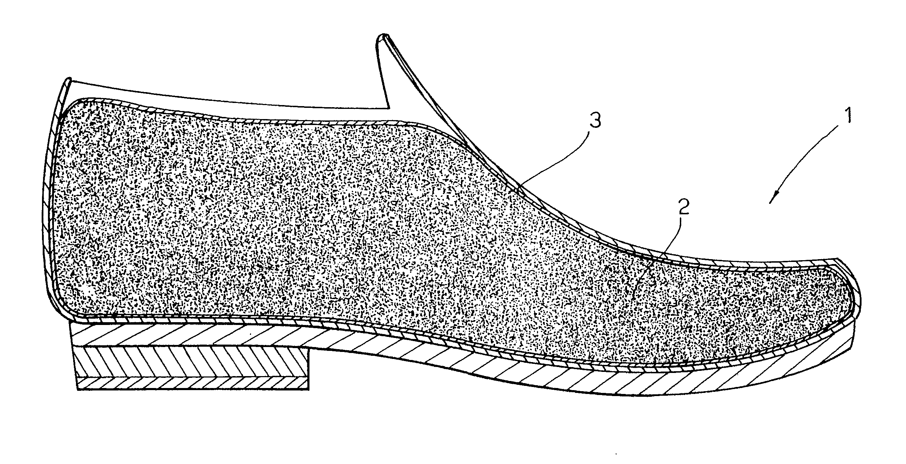

The invention will now be described in greater detail with reference to one of its purely exemplary, and therefore not restrictive embodiments, with reference to the appended figure, which illustrates an item of footwear in longitudinal section with a pack of the product according to the invention applied inside it.

With reference to this figure, an item of footwear, in particular a shoe, is shown with 1, inside of which the product 2 according to the invention has been arranged, enclosed in a pliable porous wrapping 3.

The porous wrapping 3 consists for example of cloth or of any woven or non-woven material and is foreseen in different sizes to adapt to the sizes of the footwear, in such a way that once it is arranged in the latter it substantially comes into contact with the entire inner surface. When the porous wrapping 3 containing the product according to the invention is not in use inside a shoe, it can be conserved by enclosing it hermetically, in a plastic bag for example, so as to maintain its bactericidal, fungicidal, deodorizing and adsorbent properties.

From what has been said it is easy to see the advantages connected with the use of the product according to the invention, for sanitizing, adsorbing moisture and/or perfuming footwear in general.

The product 2 according to the invention consists of a mixture of herbs or any other natural and non-natural, aromatic and medicinal product having a bactericidal and fungicidal action, with or without sprinkling with perfumes essences, and of adsorbent substances, such as silica gel, calcium chloride, sodium sulphate etc..

A typical but not restrictive formula of the product can be as follows:
- 20% thyme, 10% cinnamon, 20% origanum, 10% mint, 20% lavender, 20% silica gel.

Of course, the wrapping 3 shown in the appended figure can be replaced by a number of smaller containers, consisting for example of envelopes, small bags, pads and the like.

## Claims

1. A product for sanitizing, adsorbing moisture and/or perfuming footwear in general, characterized in that it consists of a mixture of products, natural or otherwise, with an antiseptic action, and of adsorbent substances.

2. A product according to claim 1, characterized in that perfumed essences are added to the said mixture.

3. A product according to claim 1 or 2, characterized in that said antiseptic substances are aromatic herbs, medicinal herbs and other natural or non-natural products.

4. The product according to claim 1, in which the said adsorbent substances comprise silica gel, calcium chloride, sodium sulphate and the like, alone or in combination with each other.

5. A product according to any of the previous claims, characterized by the following formula:
- 20% thyme, 10% cinnamon, 20% origanum, 10% mint, 20% lavender, 20% silica gel.

6. A product according to any one of the previous claims, in which the various component substances are mixed together in sizes varying from 2 to 20 mm.

7. A pack for containing the product according to one of the previous claims, characterized in that it consists of at least one porous wrapping or container (3) of the pliable type.

8. A pack according to claim 7, characterized in that it foresees one single wrapping (3) of a size corresponding to the size of the footwear, so as to substantially come into contact with the entire inner surface of the footwear itself.

9. A pack according to claim 7, characterized in that it comprises several containers (3) consisting of envelopes, small bags, pads and the like.

10. Method for using the product according to any one of the claims from 1 to 7, wherein said product, contained in at least one pliable porous wrapping (3), is inserted inside a shoe (1), when it is not used, so that it substantially adapts to the whole inner surface thereof.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CH-A-6 693 14   (ESMERALD S.A.)<br>* claims 1-7 *<br>– – – | 1-2 | A 61 L 9/01<br>A 43 B 17/10 |
| X | FR-A-1 602 575   (ARIES, ROBERT)<br>* the whole document *<br>– – – | 1 | |
| A | US-A-4 898 727   (KOJI OSADA ET AL.)<br>* claims 1-10 *<br>– – – | 1-10 | |
| A | US-A-2 671 277   (EVERETTE L. MONTGOMERY)<br>– – – | | |
| A | DE-A-3 215 526   (PETERS, GEB. SEBESTA, ALENKA ANTONIE)<br>– – – – – | | |
| | | | **TECHNICAL FIELDS SEARCHED (Int. Cl.5)**<br><br>A 61 L<br>A 43 B |

The present search report has been drawn up for all claims

| Place of search | Date of completion of search | Examiner |
|---|---|---|
| The Hague | 10 May 91 | ESPINOSA Y CARRETERO |